## Europäisches Patentamt
## European Patent Office
## Office européen des brevets

(19)

(11) Publication number: **0 002 904**
**B1**

(12)

# EUROPEAN PATENT SPECIFICATION

(45) Date of publication of patent specification: **08.07.81**

(51) Int. Cl.³: **A 61 C 5/02, A 61 F 13/20**

(21) Application number: **78300788.3**

(22) Date of filing: **11.12.78**

(54) **Dental probe.**

(30) Priority: **15.12.77 JP 169306/77 U**

(43) Date of publication of application:
**11.07.79 Bulletin 79/14**

(45) Publication of the grant of the European patent:
**08.07.81 Bulletin 81/27**

(84) Designated Contracting States:
**CH DE FR GB IT**

(56) References cited:
**CH - A - 367 429**
**FR - E - 58 319**
**US - A - 3 318 000**
**US - A - 3 896 802**
**US - A - 4 016 329**
**US - A - 4 044 468**

(73) Proprietor: **TEIBOW COMPANY LIMITED**
**36 Mukoujuku-cho**
**Hamamatsu-shi Shizuoka-ken (JP)**

(72) Inventor: **Yasuhiro, Aoki**
**1871 Nakagouri-cho**
**Hamamatsu-shi Shizuoka-ken (JP)**

(74) Representative: **Harrison, David Christopher et al,**
**MEWBURN ELLIS & CO 70 & 72 Chancery Lane**
**London WC2A 1AD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Dental probe

This invention relates to a probe for dental therapy and examination. More particularly, the invention relates to a device useful for dental therapy, such as removal of blood, pus and the like, wiping and applying of medicine to a tooth cavity or root canal, and for dental examination such as bacilloscopy in a tooth cavity.

Conventionally, broach cottons and paper points have been utilized for these purposes. The broach cottons are made by manually wrapping cotton fibers around the broach (a spike or rod) and then sterilizing it in a dry sterilizer. However, broach cottons have drawbacks in that the cotton fibers tend to be retained in the root canal when they are used during a dental operation and it is difficult to obtain the product in sanitary condition even by sterilization. The paper points also have drawbacks in that the preparation thereof requires a great deal of skill and much time, and the points are poor in absorbing blood, pus, liquid medicine and the like. Furthermore, it has been suggested in CH—A—367429 that an absorbent bundle of parallel fibers held together by partial adhesion might be used as a dental swab, to absorb saliva from a patient's mouth.

It is the primary object of the present examination to provide a dental therapeutic and examination probe of good quality, which can be easily inserted into the root canal or tooth cavity without leaving fibers therein and which is excellent for absorbing blood, pus, liquid medicine and the like.

The present invention provides a dental probe which combines a high absorbency for liquid, due to capillarity in the probe, with coherence, strength and sterilizability.

The probe comprises an elongate bundle of fibers extending parallel to the axis of elongation. For its use in examination or therapy, the bundle is narrowed at one or both of its ends. For strength, the fibers are secured to each other in places along their lengths so that the fiber bundle is coherent. However, the fibers define capillary passageways therebetween into which blood or pus may be drawn or in which medicines may be stored for application.

Due to its simple construction, the probe of the present invention can be produced continuously and in large quantities mechanically and therefore can be provided cheaply.

In the accompanying drawings:

Figs. 1 to 10 are schematic front views of respective embodiments of the probe having various configurations, and;

Figs. 11 to 13 are enlarged transverse cross-sectional views of embodiments of the probe, wherein the cross-sections are circular, oval and square, respectively.

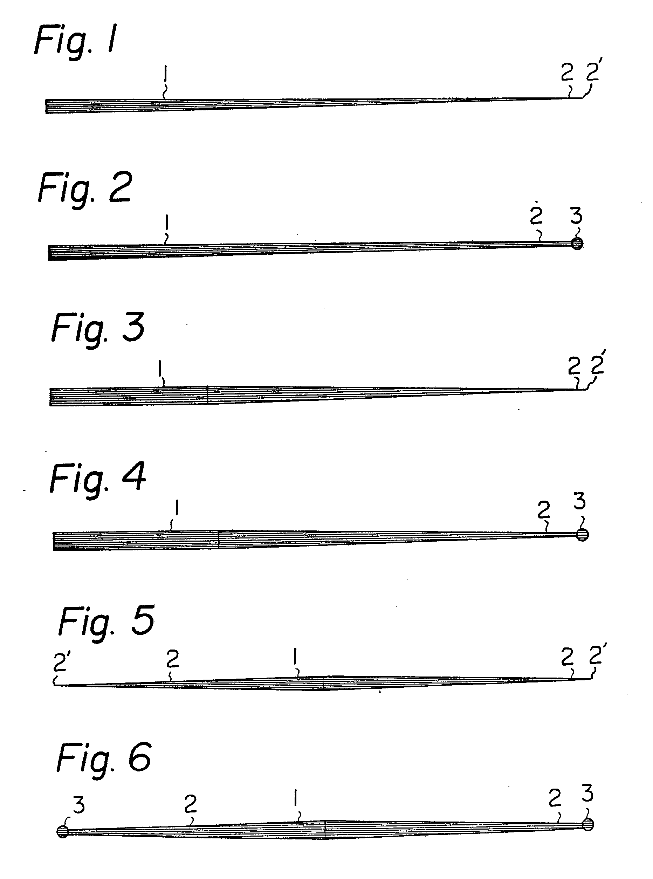

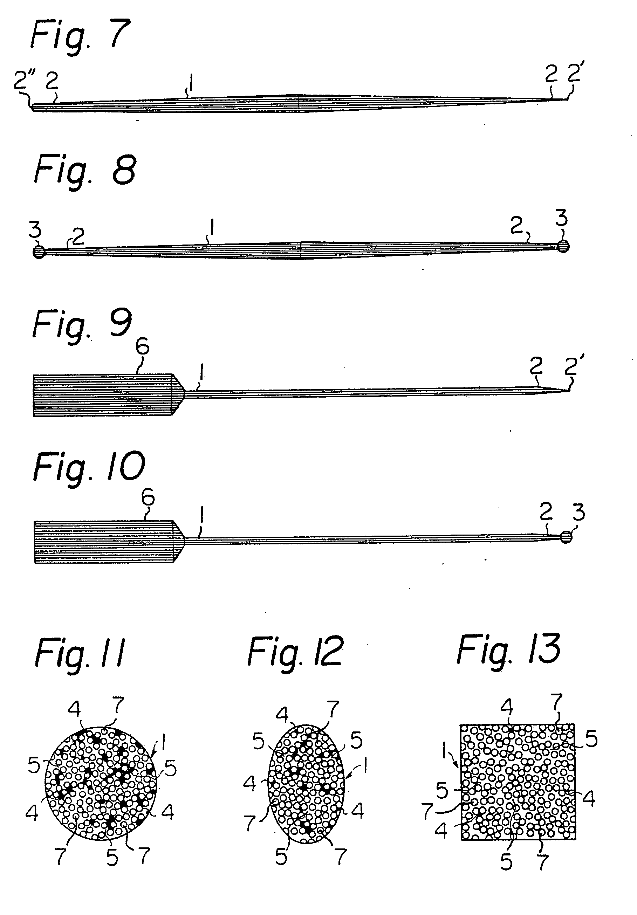

Referring now to Figs. 1 to 10, the probe of the present invention may be prepared by arranging fibers 7 in parallel, securing the fibers in that arrangement by means of an adhesive or heat fusion to form a coherent elongate bundle 1 in which, as will be described, capillary passages between the fibers are provided. The formed bundle is then cut to length and is narrowed at one or both of the longitudinal ends into a conical shape 2, 2', 2'' using, for example, a grinder, and then is sterilized. The device of the present invention so prepared can have the following dimensions, for example, the outer diameter of the elongate bundle 1 being 0.8 mm, the length of the device being 27 mm and the outer diameter of the tapered longitudinal end 2 being 0.2 mm. If desired, the tapered longitudinal end 2 of the fiber bundle 1 may be formed into sharp end 2', a blunt rounded end 2'' or a globular shape 3, during the tapering process. One end of the bundle may be of a considerably greater thickness to form a handle portion 6 (Figs. 9 and 10).

As the fibers composing the elongated bundle, any sterilizable animal, vegetable and synthetic fibers may be employed. However, synthetic fibers are preferred. Examples of synthetic fibers which can be advantageously used for the present invention are sliver-like or filamentary fibers, each having a gauge of about 3 to 20 denier, made of polyester, nylon, vinylon, polyacetal, polyurethane, polyethylene and the like. In the case of the filamentary fibers, it is desirable to employ textured fibers.

Where the fiber bundle is made coherent by an adhesive, it is advantageous that the adhesive be used in an amount as small as possible while being sufficient to maintain the configuration of the fiber bundle in a coherent, elongated rod shape, in order to produce a gentle sensation when felt by a patient and to increase the capacity of the fiber bundle for absorbing blood, pus, liquid medicine and the like, during a dental operation. Examples of a non-toxic adhesive which may be used are thermoplastic synthetic resins such as acrylic, polystyrene, polycarbonate, polyacetal, polyvinyl chloride and ABS (acrylonitrile-butadiene-styrene) resins; thermosetting resins such as unsaturated polyester, melamine, phenolic epoxide and polyurethane resins; natural thickners such as starch and casein; and CMC (carboxymethyl cellulose). As shown in Figs. 11 to 13, in the obtained device, the adhesive 4 is randomly distributed between the fibers for making the fiber bundle coherent while numerous fine openings 5 remain between the fibers communicating together in the axial direction and allowing a rapid and smooth absorption of blood, pus, liquid medicine and the like due to the capillarity of the communicating openings.

When the elongate fiber bundle is formed using synthetic fibers, the bundle can be rendered coherent due to heat fusion of the fibers. In such a case, since the fibers are generally not straight and, therefore, adjacent fibers are brought into contact with each other in some places and not in others along their lengths when the fibers are arranged in parallel, a coherent elongated fiber bundle wherein the fibers are bonded to each other in places along their lengths and across the transverse cross-section is formed if the fiber bundle is heated to such an extent that only the regions of the fibers in contact with each other are fused and bonded. Then, the fiber bundle may be formed into an appropriate configuration and sterilized as mentioned above. The device thus obtained is coherent because of the fused and bonded regions of the component fibers but possesses axially communicating numerous fine openings between the fibers, which allow a rapid and smooth absorption of blood, pus, liquid medicine and the like.

It should be noted that the fiber bundle may be formed in such a condition that the fibers in the outer portion of the bundle are secured together while the fibers in the inner portion remain more or less unsecured. Such construction may be more preferable because an increased absorption capacity thereof can be attained. Furthermore, the dental therapeutic and examination probe of the invention may have a transverse cross section which is circular, oval, triangular, tetragonal (square), polygonal or star shape. Examples are seen in Figs. 11, 12 and 13.

It has been found that the device of the present invention exhibits a liquid absorption of 40 to 60% based on the weight of the device, while conventional paper points exhibit in general a liquid absorption of only about 10%. It has also been found that the device of the invention can be thoroughly saturated with horse blood within 30 seconds but that conventional paper points can only be partially infiltrated with horse blood even for a period of 2 minutes.

The device for dental therapy and examination can be easily inserted into the interior of a root canal or tooth cavity without leaving any fibers therein, it has an excellent absorption capacity for blood, pus, liquid medicine and the like and it provides a gentle sensation when felt by a patient during a dental operation It can be produced continuously and in large quantities by mechanical production and, thus, can be provided cheaply under sanitary conditions.

## Claims

1. A fibrous axially elongate probe for dental therapy and examination in a root canal or tooth cavity characterised in that the fibers (7) of the probe extend axially in a bundle (1) which gradually narrows towards at least one end (2) the fibers (7) being secured to each other so that the bundle (1) is coherent and the fibers (7) define capillary passageways (5) between them.

2. A probe according to claim 1, wherein at least one narrowed end (2) is in a globular shape (3).

3. A probe according to claim 1 or claim 2 wherein the fibers (7) are synthetic and are secured to each other by fusion.

4. A probe according to claim 3 wherein the degree of fusion is greater at the outer portion of the bundle (1) than at the inner.

5. A probe according to claim 1 or claim 2 wherein the fibers (7) are secured to each other by an adhesive (4), the quantity of adhesive being such that only some of the openings (5) between fibers are at a given cross section of the bundle (1) occupied by adhesive.

6. A probe according to any one of the preceding claims wherein one end of the device is formed as a handle portion (6).

7. A probe according to any one of the preceding claims which is sterilized.

## Revendications

1. Sonde fibreuse allongée pour l'examen et les soins dentaires dans un canal de racine ou une cavité d'une dent caractérisée en ce que les fibres (7) de la sonde s'étendent axialement en un faisceau (1) de fibres qui s'amincit progressivement en direction d'au moins une extrémité (2), les fibres (7) étant liées les unes aux autres de façon que le faisceau soit cohérent et que les fibres (7) définissent des passages capillaires (5) entre elles.

2. Sonde selon la revendication 1, caractérisée en ce que au moins l'une de ses extrémités amincies (2) présente une forme globulaire (3).

3. Sonde selon l'une quelconque des revendications 1 et 2, caractérisée en ce que les fibres (7) sont synthétiques et liées les unes aux autres par fusion.

4. Sonde selon la revendication 3, caractérisée en ce que le degré de fusion est plus important à la partie externe du faisceau (1) que dans sa partie interne.

5. Sonde selon l'une quelconque des revendications 1 et 2, caractérisée en ce que les fibres (7) sont liées les unes aux autres su moyen d'un adhésif, la quantité d'adhésif étant telle que seulement quelques unes des ouvertures (5) définies entre les fibres dans une section donnée du faisceau (1) soient remplies par l'adhésif.

6. Sonde selon l'une quelconque des revendications 1 à 5, caractérisée en ce qu'une extrémité du dispositif est conformée en poignée (6).

7. Sonde selon l'une quelconque des revendications 1 à 6, caractérisée en ce qu'elle est stérilisée.

## Patentansprüche

1. Faserförmige, axial langgestreckte Sonde für eine Zahnbehandlung und -untersuchung in einem Wurzelkanal oder einer Zahnhöhle, dadurch gekennzeichnet, daß die Fasern (7) der Sonde sich in einem Bündel (1), das sich wenigstens zu einem Ende (2) hin allmählich verengt, axial erstrecken, die Fasern (7) derart aneinander befestigt sind, daß das Bündel (1) innerlich zusammenhält und die Fasern (7) zwischen sich kapillare Durchtrittskanäle (5) bilden.

2. Sonde nach Anspruch 1, bei der wenigstens ein verengtes Ende (2) eine kugelartige Gestalt (3) aufweist.

3. Sonde nach Anspruch 1 oder 2, bei der die Fasern (7) synthetisch und aneinander durch Verschmelzung befestigt sind.

4. Sonde nach Anspruch 3, bei der der Grad der Verschmelzung in dem äußeren Bereich des Bündels (1) größer ist als in dem inneren.

5. Sonde nach Anspruch 1 oder 2, bei der die Fasern (7) durch einen Klebstoff (4) aneinander befestigt sind, wobei die Menge des Klebstoffs derart bemessen ist, daß nur einige der Öffnungen (5) zwischen den Fasern an einem gegebenen Querschnitt des Bündels (1) durch den Klebstoff belegt sind.

6. Sonde nach einem der vorhergehenden Ansprüche, bei der ein Ende des Instruments als ein Griffbereich (6) ausgebildet ist.

7. Sonde nach einem der vorhergehenden Ansprüche, die sterilisiert ist.

*Fig. 1*

*Fig. 2*

*Fig. 3*

*Fig. 4*

*Fig. 5*

*Fig. 6*

Fig. 7

Fig. 8

Fig. 9

Fig. 10

Fig. 11

Fig. 12

Fig. 13